# EUROPÄISCHE PATENTSCHRIFT

(11) **EP 1 432 352 B1**
(45) Veröffentlichungstag und Bekanntmachung des Hinweises auf die Patenterteilung: **11.05.2005**
(21) Anmeldenummer: 02777260.7
(22) Anmeldetag: 30.09.2002
(51) Int. Cl.: A61B 5/103

(54) **VORRICHTUNG FüR DIE ANALYSE DER STÜTZREAKTION DER UNTEREN EXTREMITÄTEN ZUR DIAGNOSTIZIERUNG DES WIRBELSSAULENSUSTANDES EINES MENSCHEN**
DEVICE FOR ANALYZING THE SUPPORT REACTION OF THE LOWER EXTREMITIES FOR DIAGNOSIS OF THE STATE OF THE HUMAN SPINAL COLUMN
DISPOSITIF PERMETTANT D'ANALYSER LA REACTION DE SUPPORT DES EXTREMITES INFERIEURES D'UN ETRE HUMAIN AFIN DE DETERMINER L'ETAT DE SA COLONNE VERTEBRALE

(30) Priorität: 01.10.2001 DE 10148529
(43) Veröffentlichungstag der Anmeldung: 30.06.2004
(73) Patentinhaber: ToMetric AG, 4051 Basel (CH)
(72) Erfinder: FRANK, Zuzana, 89340 Leipheim (DE); TRETJAKOVA, Marina, Moskau 113405 (RU)
(74) Vertreter: Hentrich, Swen Dipl.-Phys. Dr.
(86) Internationale Anmeldenummer: PCT/EP2002/010933
(87) Internationale Veröffentlichungsnummer: WO 2003/028552

(56) Entgegenhaltungen:
- DE-B- 1 130 555
- FR-A- 2 491 754
- US-A- 5 025 476

## Beschreibung

Die Erfindung betrifft eine Vorrichtung für die Analyse der Stützreaktion der unteren Extremitäten zur Diagnostizierung des Wirbelsäulenzustandes eines Menschen, mit einer rechten Stützbühne und einer linken Stützbühne, die jeweils zumindest höhenverstellbar und um zwei horizontale Achsen drehbar gelagert sind und eine Standfläche aufweisen.

Der Wirbelsäule als zentrales tragendes Element des Skelettes kommt eine wesentliche Rolle bei der Beurteilung des Gesundheitszustandes eines Menschen zu, wobei Fehlstellungen einzelner Wirbel und daraus resultierend ein von der natürlichen Lage und Krümmung abweichender Wirbelsäulenzustand auf vielfältige Weise die Gesundheit eines Menschen beeinträchtigen können. Einige Krankheitsbilder, wie beispielsweise Schwindelattacken, haben vordergründig nichts mit der Wirbelsäule zu tun, können aber doch vertebragen bedingt sein, wenn beispielsweise die im Zwischenwirbelraum positionierten Bandscheiben auf die Nerven des Rückenmarkes drücken. Neben der richtigen Ausrichtung benachbarter Wirbel relativ zueinander ist auch die Wirbelsäule als Gesamtheit zu betrachten zur Beurteilung von deren Skoliose. Ihre tragende Funktion kann die Wirbelsäule aber nur im Zusammenwirken mit den unteren Extremitäten ausüben, die sämtliche Last bei der normalen natürlichen Haltung und Fortbewegung des Menschen aufnehmen müssen. Fehlstellungen der Wirbelsäule können verursacht sein durch eine unterschiedliche Länge der beiden Beine des Menschen, der zum Erzielen eines aufrechten Ganges die abweichende Länge seiner Beine durch eine entsprechende Haltung seines Oberkörpers zu kompensieren versucht. Umgekehrt kann aber auch eine scheinbar abweichende Länge der beiden Beine durch eine von der Wirbelsäule verursachte Schiefstellung des Beckens gegeben sein. Im Ergebnis bedarf es einer detaillierten und sorgfältigen Analyse der Kraftübertragungskette von den unteren Extremitäten zur Wirbelsäule mit den darin involvierten Gelenken, um Fehlstellungen der Wirbelsäule ausgleichen und deren natürliche Lage erreichen zu können.

Zur Diagnostizierung des Wirbelsäulenzustandes ist bereits vorgeschlagen worden, unterschiedliche Beinlängen dadurch auszugleichen, daß man Stützbühnen mit unterschiedlicher Höhenlage vorgibt. Dies hat sich allerdings als nicht ausreichend gezeigt und nicht zu zufriedenstellenden Ergebnissen geführt, da die weiteren biomechanischen Folgen der unterschiedlichen Höhenlage der Stützbühnen unberücksichtigt geblieben sind, nämlich daß die Anhebung eines Beines zwingend mit einer Rotation des dieses Bein tragenden Fußes im Knöchelgelenk verbunden ist, die bisher weder qualitativ noch quantitativ ausgewertet werden konnte. Dazu sind auch nicht Vorrichtungen in der Anlage, die allein zur Untersuchung oder für das Training des Knöchelgelenks konzipiert sind, wie beispielsweise in der US 6,162,189 beschrieben.

Eine Vorrichtung der eingangs genannten Art ist in der DE-AS 1 130 555 beschrieben, mit der reproduzierbar die relative Stellung der Stützbühnen und deren Neigung vom Arzt eingestellt werden kann nach der subjektiven Beurteilung des Arztes hinsichtlich der Korrektur der Haltung des untersuchten Menschens.

Die US 5 025 476 offenbart eine Vorrichtung zur Analyse der Gestalt eines Fußabdruckes unter Verwendung einer durchsichtigen Standfläche, durch die der Fußabdruck mittels einer Kamera als Moiré-Bild erfaßt und in einem Computer ausgewertet werden kann. Da die Standfläche waagerecht und starr ist, ist die Vorrichtung nicht zur Untersuchung der Stützreaktion oder des Wirbelsäulenzustandes geeignet.

Der Erfindung liegt daher die Aufgabe zugrunde, eine Vorrichtung der eingangs genannten Art so auszubilden, daß die Stützreaktion der unteren Extremitäten bewertet und charakterisiert werden kann.

Diese Aufgabe wird bei einer vorrichtung der eingangs genannten Art dadurch gelöst, daß die Standflächen durchsichtig sind, daß unter jeder Standfläche eine erste elektronische Einheit zur optischen Erfassung der plantaren Oberfläche des der jeweiligen Stützbühne zugeordneten Fußes des Menschen angeordnet ist, daß jede Einheit mit einem Computersystem verbunden ist, und daß die Stützbühnen gemeinsam in einer Plattform auf einem Drehungsmodul angeordnet sind, das um eine symmetrisch zwischen den beiden Stützbühnen verlaufende vertikale Achse drehbar gelagert ist.

Durch diese Vorrichtung ergibt sich nun erstmals die Möglichkeit, neben der Höhenlage der rechten und der linken Stützbühne bzw. deren Differenz auch den Schrägungswinkel des Fußes in dessen Längsrichtung und der dazu senkrechten Richtung zu bestimmen und zwar in einem vorgegebenen und reproduzierbaren Zustand, für den der Kontakt der plantaren Oberfläche des Fußes mit der Standfläche charakteristisch ist, insbesondere durch eine vollständige Auflage, also bei einer maximal erreichbaren Kontaktfläche, die mittels der Einheit gemessen und im Computersystem protokolliert und ausgewertet werden, insbesondere zur Bestimmung von Änderungen bei Modifikationen der Körperhaltung. Weiterhin ermöglicht diese Ausführungsform auch eine Drehung der unteren Extremitäten um die Körperlängsachse, so daß ein weiterer Parameter variiert und bestimmt werden kann zur Definition des Wirbelsäulenzustandes, also auch die Beweglichkeit in der Hüfte bzw. eine Drehung des Oberkörpers berücksichtigt wird.

Eine besonders bevorzugte Ausführungsform der Erfindung ist dadurch gekennzeichnet, daß jede Stützbühne auf einer Antriebsbühne um drei senkrecht zueinander stehende Achsen drehbar gelagert und in Richtung dieser Achsen linear verstellbar ist. Diese Ausführungsform mit einer erweiterten Verstellmöglichkeit der Stützbühnen in insgesamt sechs Freiheitsgraden ermöglicht eine bessere Anpassung an die vorliegenden anatomischen Gegebenheiten des Menschen, insbesondere eine Beeinflussung von dessen Standbreite, die in Abhängigkeit der Körpergröße des Menschen und insbesondere von dessen Schulterbreite gewählt werden sollte.

Es hat sich als zweckmäßig erwiesen, daß zur Verstellung der Stützbühnen sowie des Drehungsmoduls kraftbetätigbare Aktuatoren vorgesehen sind, die mit dem Computersystem zu ihrer Kontrolle und Steuerung verbunden sind. Derartige Aktuatoren zur Verstellung der Stützbühne gegenüber der Antriebsbühne in sechs Freiheitsgraden sind aus der US 5,987,726 bekannt und müssen daher hier nicht weiter erläutert werden. Zu betonen ist lediglich der Vorteil der Einbindung dieser kraftbetätigbaren Aktuatoren an das Computersystem im Hinblick auf einen automatisierten Ablauf bei der Untersuchung des wirbelsäulenzustandes.

Es hat sich als zweckmäßig erwiesen, wenn die optische Einheit durch einen Scanner oder eine Digitalkamera oder eine Videokamera gebildet ist, die in vielfältigen Ausführungsformen kommerziell erhältlich sind und ohne Probleme eine Integration in das Computersystem ermöglichen.

Um aussagekräftige Untersuchungsergebnisse zu erhalten, ist es günstig, wenn jeder Stützbühne eine mit dem Computersystem verbundene Waage zugeordnet ist, da so die Möglichkeit besteht, unabhängig von dem Gleichgewichtsgefühl des Menschen sowie dessen Fähigkeit zur Kooperation zu bestimmen, ob eine gleichmäßige Belastung beider Stützbühnen erfolgt.

Vorteilhaft ist es weiterhin, wenn das Computersystem neben dem Monitor für den Operator einen Informationsmonitor für den Menschen aufweist, da so die Fähigkeit und Bereitschaft des Menschen zur Kooperation gesteigert werden kann und zugleich eine unmittelbare Rückkopplung an den Menschen über dessen momentane Stellung und vorzunehmende Variationen zum Erreichen der gewünschten Stehposition möglich ist. Der Informationsmonitor ist an einer vor den Stützbühnen mittig angeordneten, höhenverstellbaren Säule angeordnet, um so eine Anpassung der Lage des Informationsmonitors an die Größe des Menschen vornehmen zu können und diesem eine Beobachtung des Informationsmonitores ohne Drehung des Kopfes in der Halswirbelsäule oder anderer Teile der Wirbelsäule zu ermöglichen.

Zweckmäßigerweise ist der Säule ein Schutzgeländer zugeordnet, das zur Verhinderung eines Gleichgewichtsverlustes des Menschen genutzt werden kann. Bei dieser Ausführungsform ist es vorteilhaft, wenn dem Schutzgeländer eine mit dem Computersystem verbundene Waage zugeordnet ist, damit bei der Analyse des Wirbelsäulenzustandes sicher gestellt werden kann, daß über das Schutzgeländer keine nennenswerte Abstützung des Menschen erfolgt, sondern dessen Gewicht nahezu vollständig auf den beiden Stützbühnen ruht.

Um eine definierte Ausgangsposition für den Start der Untersuchungen zu erhalten, sind auf jeder Standfläche Leitschienen für die lagerichtige Orientierung der Füße des Menschen angeordnet, insbesondere dergestalt, daß die Leitschienen zur Anlage an der Fersenzone und der Innenzone eines jeden Fußes vorgesehen sind und einen Winkel der Fußlängsachsen von 12° bis 20°, vorzugsweise von 16° vorgeben.

Um den Zeitbedarf für die Durchführung der Untersuchung zu reduzieren und möglichst schnell den Zustand einer gleichmäßigen Belastung der beiden Stützbühnen erreichen zu können, ist eine Signaleinrichtung zur Anzeige der relativen Belastung der Stützbühnen, insbesondere zur Anzeige von deren gleichmäßiger Belastung vorgesehen, die zweckmäßigerweise dem Informationsmonitor zugeordnet ist und so dem Menschen ermöglicht, selber die Gewichtsverteilung den Erfordernissen anzupassen.

Zur Protokollierung der Untersuchung und automatisierten Auswertung ist eine zweite elektronische Einheit zur optischen Erfassung des Wirbelsäulenzustandes des Menschen vorgesehen, die bei Anbindung an das Computersystem auch eine Wiedergabe des aufgenommenen Bildes der Wirbelsäule auf dem Informationsmonitor ermöglicht.

Jeder Standfläche ist eine Mehrzahl von Drucksensoren zur ortsaufgelösten Erfassung des Druckes in der Kontaktfläche zwischen Fuß und Standfläche zugeordnet, die die durch die erste elektronische Einheit zur optischen Erfassung gewonnene Information ergänzen und absichern.

Im folgenden wird die Erfindung an in der Zeichnung dargestellten Ausführungsbeispielen näher erläutert; es zeigen:
- Fig. 1: eine Draufsicht auf eine erste Ausführungsform der erfindungsgemäßen Vorrichtung,
- Fig. 2: eine Vorderansicht der Vorrichtung aus Fig. 1,
- Fig. 3: eine Draufsicht auf die Stützbühne,
- Fig. 4: eine Draufsicht auf die Antriebsbühne,
- Fig. 5: eine Seitenansicht mit den zwischen der Antriebsbühne und der Stützbühne angeordneten Aktuatoren,

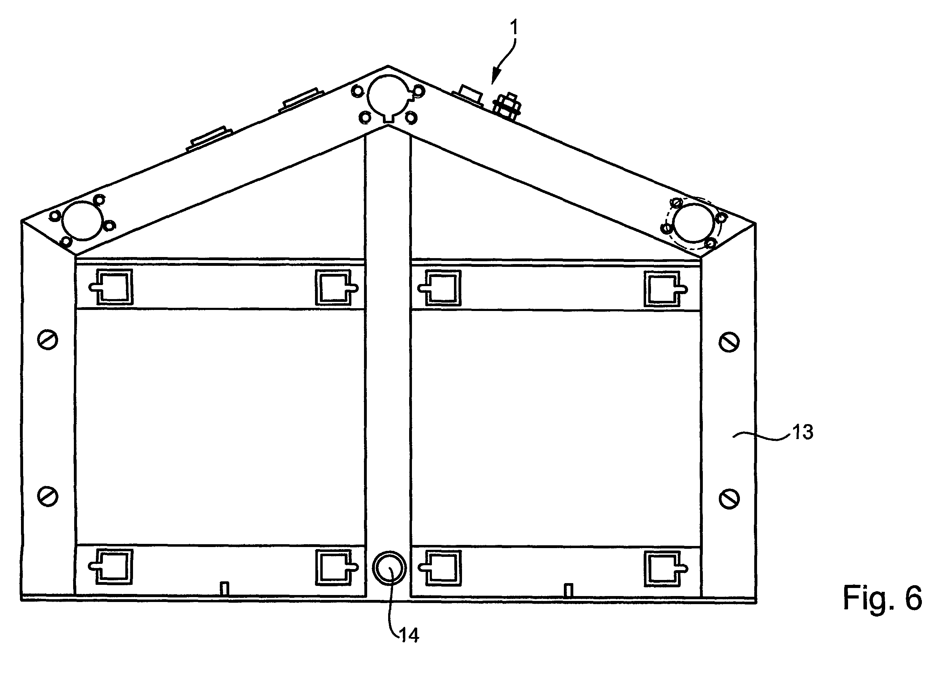

- Fig. 6: eine Draufsicht auf das Drehungsmodul, und
- Fig. 7: eine schematische Darstellung einer zweiten Ausführungsform.

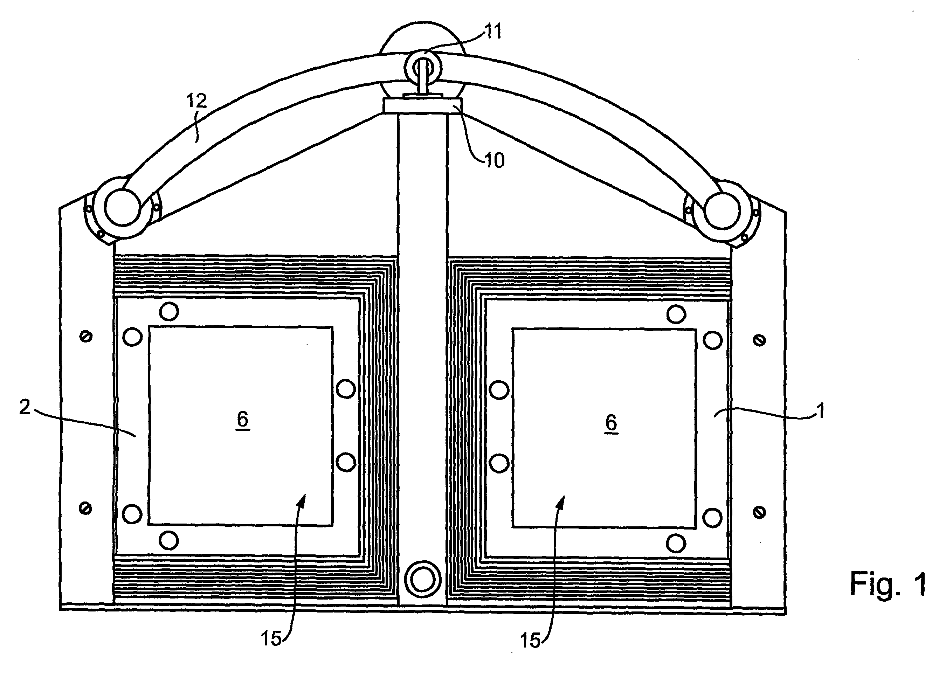

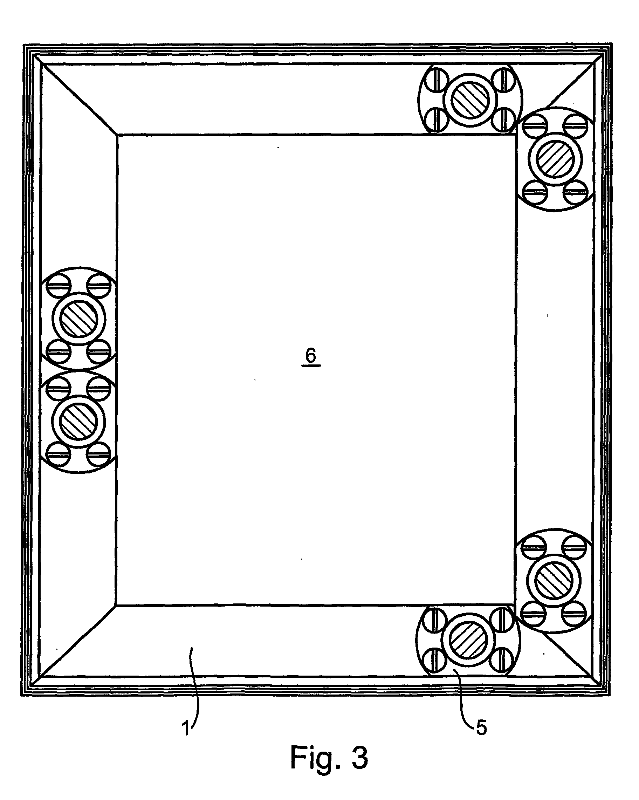

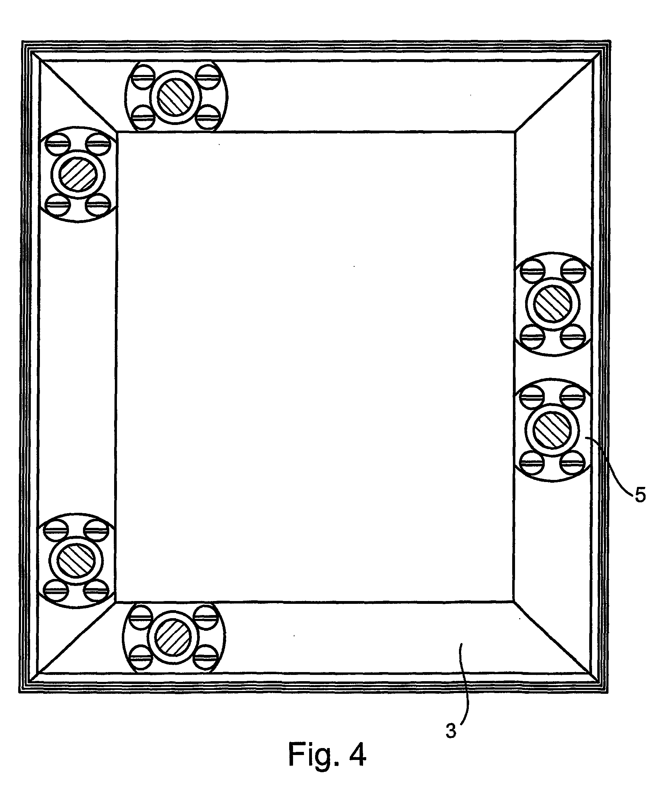

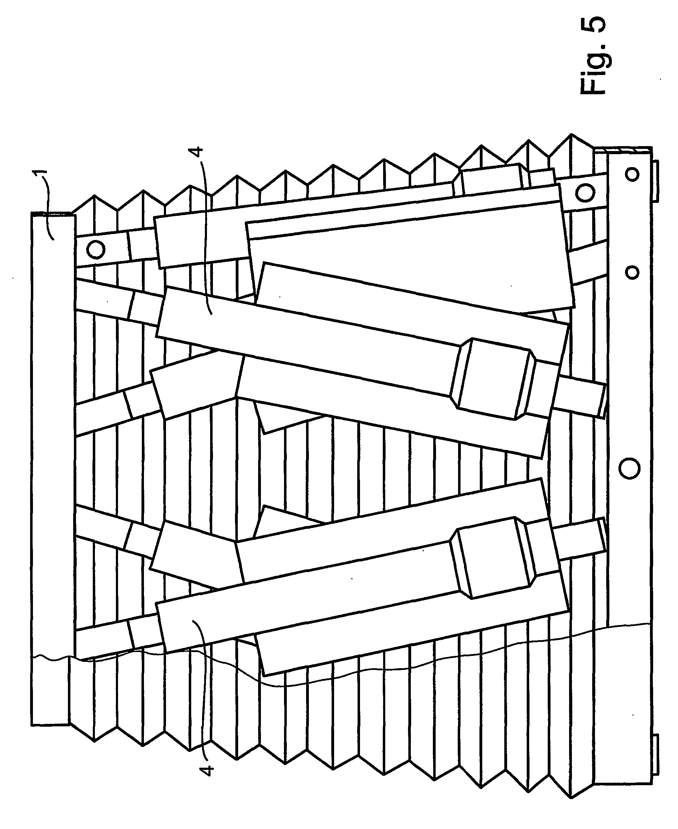

In der Fig. 1 ist eine Vorrichtung gezeigt, die für die Analyse der Stützreaktion der unteren Extremitäten eines Menschen zur Diagnostizierung von dessen Wirbelsäulenzustandes dient. Diese Vorrichtung weist eine rechte Stützbühne 1 sowie eine linke Stützbühne 2 auf, die jeweils auf einer Antriebsbühne 3 gelagert sind und sechs Bewegungsfreiheitsgrade aufweisen, nämlich um drei senkrecht zueinanderstehende Achsen verdreht werden können und in Richtung dieser Achsen linear verstellbar sind. Zur Verstellung der Stützbühnen 1, 2 sind kraftbetätigbare Aktuatoren 4 vorgesehen, die in der Fig. 5 sichtbar dargestellt sind, während in der Fig. 1 sowie den Fig. 3 und 4 lediglich deren Anschlüsse 5 an die Stützbühne 1 und die Antriebsbühne 3 ersichtlich sind.

Jede Stützbühne 1, 2 weist eine durchsichtige Standfläche 6 auf, unter der eine elektronische Einheit 15 zur optischen Erfassung der plantaren Oberfläche des der jeweiligen Stützbühne 1, 2 zugeordneten Fußes des Menschen angeordnet ist. Zur Realisierung der optischen Einheit ist sowohl ein Scanner als auch eine Digital- oder eine Videokamera geeignet, wobei sich der Begriff "durchsichtig" auf den von der optischen Einheit erfaßten Spektrumsteil bezieht, der beispielsweise bei Verwendung einer Wärmebildkamera dem IR-Bereich entsprechen kann.

Die Vorrichtung ist weiterhin dadurch ergänzt, daß jeder Stützbühne 1, 2 eine Waage zugeordnet ist, durch die die Belastung jeder Stützbühne 1, 2 ermittelt werden kann.

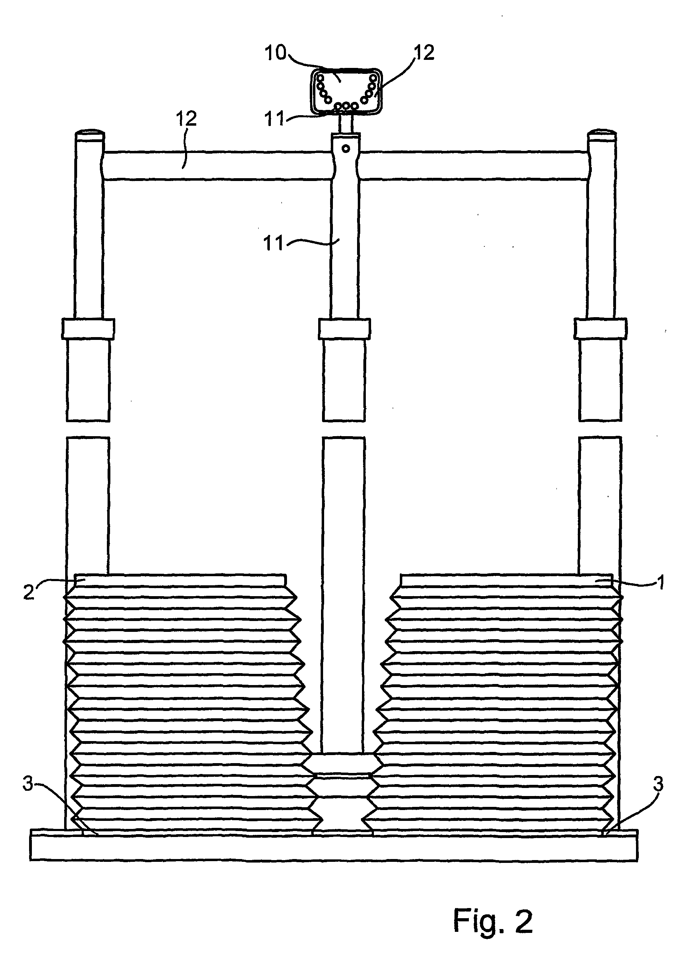

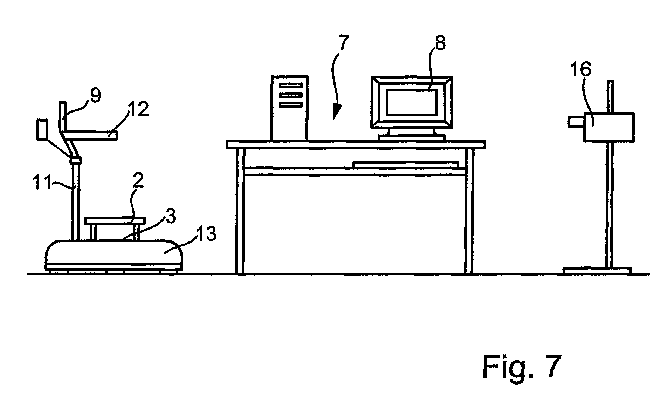

Sowohl die optische Einheit 15 als auch die Aktuatoren 4 und die Waagen sind mit einem Computersystem 7 verbunden, das neben dem Monitor 8 für den Operator ein Informationsmonitor 9 für den Menschen aufweist, um eine direkte Rückkopplung über die Wirkungen seiner Handlungen zu ermöglichen (Fig. 7). Fig. 2 zeigt eine Ausführungsform, bei der der Vorrichtung eine Signaleinrichtung 10 zur Anzeige der relativen Belastung der Stützbühnen 1, 2 zugeordnet ist, wobei diese Signaleinrichtung 10 in dem gezeigten Ausführungsbeispiel durch eine Mehrzahl von Signallampen 11, 12 gebildet ist, die teilweise (12) einer der beiden Stützbühnen 1, 2 zugeordnet sind und aufleuchten, wenn die Belastung dieser Stützbühne 1, 2 größer als die Belastung der anderen Stützbühne 1, 2 ist. Ein Teil der Signallampen 11 ist zur Kennzeichnung einer gleichmäßigen Belastung vorgesehen. Die Signaleinrichtung 10 ist an einer vor den Stützbühnen 1, 2 mittig angeordneten, höhenverstellbaren Säule 11 angeordnet, der weiterhin ein Schutzgeländer 12 zugeordnet ist, das eine mit dem Computersystem 7 verbundene weitere Waage aufweist.

Die Vorrichtung verfügt über ein gleichfalls durch Aktuatoren, die mit dem Computersystem 7 verbunden sind, verstellbares Drehungsmodul 13, in dem die Stützbühnen 1, 2 gemeinsam in einer Plattform angeordnet sind, wobei das Drehungsmodul 13 um eine symmetrisch zwischen den beiden Stützbühnen 1, 2 verlaufende vertikale Achse 14 drehbar gelagert ist.

Die Verwendung dieser Vorrichtung zur Analyse der Stützreaktion der unteren Extremitäten erfolgt dergestalt, daß zunächst der Mensch sich auf die Stützbühne 1, 2 stellt, wobei er das Schutzgeländer 12 ergreifen kann, um einem Verlust des Gleichgewichtes vorzubeugen. Zu dieser Startposition wird mittels der ersten optischen Einheit 15 ein Bild der plantaren Oberfläche der Füße aufgenommen und zum Vergleich im Computersystem 7 gespeichert. In Interaktion mit dem Operator oder automatisiert kann eine oder auch beide Stützbühnen 1, 2 verstellt werden, insbesondere deren Höhenlage verändert werden, um ausgehend von dem bei dem Menschen vorliegenden Wirbelsäulenzustand eine Veränderung vorzunehmen und eine natürliche Stellung der Wirbelsäule zu erzielen. Über die Abbildung der plantaren Oberfläche beider Füße wird kontrolliert, wie der Mensch auf der Standfläche 6 der Stützbühnen 1, 2 auffußt, um bei dann weiterhin gegebener gleichmäßiger Belastung der Stützbühnen 1, 2, die mittels der Waagen über die Signaleinrichtung 10 kontrolliert werden kann, die die Raumorientierung der Stützbühnen 1, 2 kennzeichnenden Parameter und damit die Stützreaktion der unteren Extremitäten zu bestimmen. Über eine zweite elektronische Einheit 16 zur optischen Erfassung des Wirbelsäulenzustandes, beispielsweise der in Fig. 7 dargestellten Videokamera, kann der Zustand der Wirbelsäule erfaßt werden, um nachfolgend Vorschläge für eine Therapie unterbreiten zu können, mit der dauerhaft durch Einlagen, orthopädische Schuhe oder dergleichen der gewünschte Wirbelsäulenzustand erreicht werden kann.

## Patentansprüche

1. Vorrichtung für die Analyse der Stützreaktion der unteren Extremitäten zur Diagnostizierung des Wirbelsäulenzustandes eines Menschen, mit einer rechten Stützbühne (1) und einer linken Stützbühne (2), die jeweils zumindest höhenverstellbar und um zwei horizontale Achsen drehbar gelagert sind und eine Standfläche (6) aufweisen,
**dadurch gekennzeichnet, daß** die Standflächen (6) durchsichtig sind, daß unter jeder Standfläche (6) eine elektronische Einheit (15) zur optischen Erfassung der plantaren Oberfläche des der jeweiligen Stützbühne (1, 2) zugeordneten Fußes des Menschen angeordnet ist, daß jede Einheit (15) mit einem Computersystem (7) verbunden ist, und daß die Stützbühnen (1, 2) gemeinsam in einer Plattform auf einem Drehungsmodul (13) angeordnet sind, das um eine symmetrisch zwischen den beiden Stützbühnen (1, 2), verlaufende vertikale Achse (14) drehbar gelagert ist.

2. Vorrichtung nach Anspruch 1, **dadurch gekennzeichnet, daß** jede Stützbühne (1, 2) auf einer Antriebsbühne (3) um drei senkrecht zueinander stehende Achsen drehbar gelagert und in Richtung dieser Achsen linear verstellbar ist.

3. Vorrichtung nach Anspruch 1 oder 2, **dadurch gekennzeichnet, daß** zur Verstellung der Stützbühnen (1, 2) sowie des Drehungsmoduls (13) kraftbetätigbare Aktuatoren (4) vorgesehen sind, die mit dem Computersystem (7) zu ihrer Kontrolle und Steuerung verbunden sind.

4. Vorrichtung nach Anspruch 1, **dadurch gekennzeichnet, daß** die optische Einheit (15) durch einen Scanner oder eine Digitalkamera oder eine Videokamera gebildet ist.

5. Vorrichtung nach einem der Ansprüche 1 bis 4, **dadurch gekennzeichnet, daß** jeder Stützbühne (1, 2) eine mit dem Computersystem (7) verbundene Waage zugeordnet ist.

6. Vorrichtung nach einem der Ansprüche 1 bis 5, **dadurch gekennzeichnet, daß** das Computersystem (7) neben dem Monitor (8) für den Operator einen Informationsmonitor (9) für den Menschen aufweist.

7. Vorrichtung nach Anspruch 6, **dadurch gekennzeichnet, daß** der Informationsmonitor (9) an einer vor den Stützbühnen (1, 2) mittig angeordneten, höhenverstellbaren Säule (11) angeordnet ist.

8. Vorrichtung nach Anspruch 7, **dadurch gekennzeichnet, daß** der Säule (11) ein Schutzgeländer (12) zugeordnet ist.

9. Vorrichtung nach Anspruch 8, **dadurch gekennzeichnet, daß** dem Schutzgeländer (12) eine mit dem Computersystem (7) verbundene Waage zugeordnet ist.

10. Vorrichtung nach einem der Ansprüche 1 bis 9, **dadurch gekennzeichnet, daß** auf jeder Standfläche (6) Leitschienen für die lagerichtige Orientierung der Füße des Menschen angeordnet sind.

11. Vorrichtung nach Anspruch 10, **dadurch gekennzeichnet, daß** die Leitschienen zur Anlage an der Fersenzone und der Innenzone eines jeden Fußes vorgesehen sind und einen Winkel der Fußlängsachsen von 12° bis 20°, vorzugsweise von 16° vorgeben.

12. Vorrichtung nach einem der Ansprüche 5 bis 11, **dadurch gekennzeichnet, daß** eine Signaleinrichtung (10) zur Anzeige der relativen Belastung der Stützbühnen (1, 2), insbesondere zur Anzeige von deren gleichmäßiger Belastung vorgesehen ist.

13. Vorrichtung nach Anspruch 11, **dadurch gekennzeichnet, daß** die Signaleinrichtung (10) dem Informationsmonitor (9) zugeordnet ist.

14. Vorrichtung nach einem der Ansprüche 1 bis 13, **dadurch gekennzeichnet, daß** eine zweite elektronische Einheit (16) zur optischen Erfassung des Wirbelsäulenzustandes des Menschen vorgesehen ist.

15. Vorrichtung nach einem der Ansprüche 1 bis 14, **dadurch gekennzeichnet, daß** jeder Standfläche (6) eine Mehrzahl von Drucksensoren zur ortsaufgelösten Erfassung des Druckes in der Kontaktfläche zwischen Fuß und Standfläche (6) zugeordnet ist.

## Claims

1. Device for analysing the support response of the lower extremities, for the purpose of diagnosing the state of a person's spine, with a right support platform (1) and a left support platform (2) which are respectively at least height-adjustable and are pivoted around two horizontal axes, and have a standing surface (6),
**characterised in that**
the standing surfaces (6) are transparent, that an electronic unit (15) for the optical recording of the plantar surface of the person's foot assigned to the respective support platform (1, 2) is arranged under each standing surface (6), that each unit (15) is connected to a computer system (7), and that the support platforms (1, 2) are jointly arranged in a platform on a rotation module (13) that is pivoted around a vertical axis (14) that runs symmetrically between the two support platforms (1, 2).

2. Device in accordance with claim 1, **characterised in that** each support platform (1, 2) is pivoted on a drive platform (3) around three axes that stand perpendicular to one another, and is adjustable in a linear manner in the direction of these axes.

3. Device in accordance with claim 1 or 2, **characterised in that** to adjust the support platforms (1, 2) and the rotation module (13), power-operated actuators (4) are provided, which are connected to the computer system (7), for their monitoring and control.

4. Device in accordance with claim 1, **characterised in that** the optical unit (15) is formed by a scanner or a digital camera or a video camera.

5. Device in accordance with one of the claims 1 to 4, **characterised in that** assigned to each support platform (1, 2) are scales that are connected to the computer system (7).

6. Device in accordance with one of the claims 1 to 5, **characterised in that** the computer system (7) has, besides the monitor (8) for the operator, an information monitor (9) for the person.

7. Device in accordance with claim 6, **characterised in that** the information monitor (9) is arranged on a height-adjustable column (11) that is arranged centrally in front of the support platforms (1, 2).

8. Device in accordance with claim 7, **characterised in that** a guard rail (12) is assigned to the column (11).

9. Device in accordance with claim 8, **characterised in that** assigned to the guard rail (12) are scales that are connected to the computer system (7).

10. Device in accordance with claims 1 to 9, **characterised in that** arranged on each standing surface (6) are guide rails for the correct orientation of the person's feet

11. Device in accordance with claim 10, **characterised in that** the guide rails are envisaged to rest against the heel zone and the inner zone of each foot, and set an angle of the longitudinal axes of the feet of 12° to 20°, preferably 16°.

12. Device in accordance with one of the claims 5 to 11, **characterised in that** a signal device (10) is provided, to display the relative loading of the support platforms (1, 2), in particular to display their even loading.

13. Device in accordance with claim 11, **characterised in that** the signal device (10) is assigned to the information monitor (9).

14. Device in accordance with one of the claims 1 to 13, **characterised in that** a second electronic unit (16) is provided, for the optical recording of the state of the person's spine.

15. Device in accordance with one of the claims 1 to 14, **characterised in that** assigned to each standing surface (6) are a number of pressure sensors for the spatially resolved recording of the pressure in the contact area between the foot and the standing surface (6).

## Revendications

1. Dispositif pour l'analyse de la réaction de support des extrémités inférieures pour le diagnostic de l'état de la colonne vertébrale d'un homme, comportant un plateau de support (1) droit et un plateau de support (2) gauche, qui tous deux sont réglables en hauteur, peuvent pivoter autour de deux axes horizontaux et présentent une surface d'appui (6), **caractérisé par le fait que** les surfaces d'appui (6) sont transparentes, **par le fait que** sous chaque surface d'appui (6) est disposée une unité (15) électronique de palpage optique de la surface plantaire du pied humain associé au plateau de support (1, 2) concerné, **par le fait que** chaque unité (15) est reliée à un système d'ordinateur (7) et **par le fait que** les plateaux de support (1, 2) sont disposés ensemble dans une plateforme sur un module de rotation (13), qui est monté tournant autour d'un axe vertical (14) positionné symétriquement entre les deux plateaux de support (1, 2).

2. Dispositif selon la revendication 1, **caractérisé par le fait que** chaque plateau de support (1, 2) est monté sur un plateau d'entraînement (3) avec possibilité de rotation autour de trois axes perpendiculaires entre eux et avec possibilité de déplacement linéaire dans la direction desdits axes.

3. Dispositif selon la revendication 1 ou 2, **caractérisé par le fait qu'**il est prévu pour le réglage des plateaux de support (1, 2) ainsi que du module de rotation (13) des actionneurs (4) mus par une source d'énergie extérieure qui sont reliés au système d'ordinateur (7) à des fins de contrôle et de commande.

4. Dispositif selon la revendication 1, **caractérisé par le fait que** l'unité optique (15) est formée d'un scanner ou d'une caméra numérique ou d'une caméra vidéo.

5. Dispositif selon une des revendications 1 à 4, **caractérisé par le fait qu'**à chaque plateau de support (1, 2) est associée une balance reliée au système d'ordinateur (7).

6. Dispositif selon une des revendications 1 à 5, **caractérisé par le fait que** le système d'ordinateur (7) comprend en plus du moniteur (8) pour l'opérateur un moniteur d'information (9) pour l'homme.

7. Dispositif selon la revendication 6, **caractérisé par le fait que** le moniteur d'information (9) est disposé sur une colonne (11) réglable en hauteur, disposée de manière centrale devant les plateaux de support (1, 2).

8. Dispositif selon la revendication 7, **caractérisé par le fait qu'**une rampe de protection (12) est associée à la colonne (11).

9. Dispositif selon la revendication 8, **caractérisé par le fait qu'**à la rampe de protection (12) est associée une balance reliée au système d'ordinateurs (7).

10. Dispositif selon une des revendications 1 à 9, **caractérisé par le fait que** sur chaque surface d'appui (6) sont disposées des règles de guidage pour le positionnement correct des pieds de l'homme.

11. Dispositif selon la revendication 10, **caractérisé par le fait que** les règles de guidage sont prévues pour le contact avec la région du talon et la région intérieure de chaque pied et imposent un angle entre les axes longitudinaux des pieds compris entre 12° et 20°, de préférence de 16°.

12. Dispositif selon une des revendications 5 à 11, **caractérisé par le fait qu'**il est prévu un système de signalisation (10) pour indiquer la charge relative des plateaux de support (1, 2), plus particulièrement pour indiquer la charge régulière de ceux-ci.

13. Dispositif selon la revendication 11, **caractérisé par le fait que** le système de signalisation (10) est associé au moniteur d'information (9).

14. Dispositif selon une des revendications 1 à 13, **caractérisé par le fait qu'**il est prévu une deuxième unité électronique (16) pour la détection optique de l'état de la colonne vertébrale de l'homme.

15. Dispositif selon une des revendications 1 à 14, **caractérisé par le fait qu'**à chaque surface d'appui (6) est associée une pluralité de capteurs de pression pour enregistrer avec une résolution locale la pression au niveau de la surface de contact entre le pied et la surface d'appui (6).
